# EUROPÄISCHE PATENTSCHRIFT

(11) **EP 4 413 645 B1**
(45) Veröffentlichungstag und Bekanntmachung des Hinweises auf die Patenterteilung: **30.09.2026**
(21) Anmeldenummer: 22800614.4
(22) Anmeldetag: 04.10.2022
(51) Int. Cl.: H02J 7/00, A61F 9/008

(54) **OPHTHALMOLOGISCHES LASERTHERAPIESYSTEM MIT EINEM ZWISCHENSPEICHER ZUR ZYKLISCHEN LEISTUNGSGLÄTTUNG**
OPHTHALMOLOGICAL LASER THERAPY SYSTEM HAVING AN INTERMEDIATE STORAGE UNIT FOR CYCLICAL POWER SMOOTHING
SYSTÈME DE TRAITEMENT OPHTALMOLOGIQUE PAR LASER COMPRENANT UN ACCUMULATEUR INTERMÉDIAIRE POUR LE LISSAGE DE PUISSANCE CYCLIQUE

(30) Priorität: 05.10.2021 DE 102021211227
(43) Veröffentlichungstag der Anmeldung: 14.08.2024
(73) Patentinhaber: Carl Zeiss Meditec AG, 07745 Jena (DE)
(72) Erfinder: STOBRAWA, Gregor, 07745 Jena (DE); WEIMER, Wolf, 07745 Jena (DE); FEIGE, Torsten, 07745 Jena (DE); KNAUER, Andreas, 07745 Jena (DE); KRAUSE, Tobias, 07745 Jena (DE)
(74) Vertreter: Nieten, Christoph
(86) Internationale Anmeldenummer: PCT/EP2022/077510
(87) Internationale Veröffentlichungsnummer: WO 2023/057416

(56) Entgegenhaltungen:
- DE-A1- 102018 200 017
- KR-A- 20160 008 313
- US-A1- 2008 269 729
- US-A1- 2019 044 336

## Beschreibung

Die vorliegende Erfindung betrifft einen Zwischenspeicher für ein ophthalmologisches Lasertherapiesystem mit einer Leistungsaufnahme von bis zu 1600W, das ein elektrisches Lastelement aufweist. Die Erfindung betrifft weiterhin ein ophthalmologisches Lasertherapiesystem mit einem Zwischenspeicher.

Ophthalmologische Lasertherapiesysteme sind komplexe elektromechanische Anordnungen, die unterschiedliche Komponenten aufweisen. Diese Komponenten sind elektrische Verbraucher, die eine entsprechende elektrische Last darstellen. Bei solchen Lastelementen kann es sich beispielsweise um Computer, Benutzerschnittstellen wie Monitore, um Steuergeräte oder um Aktoren für mechanische Bewegungen sowie um die Laserquelle selbst und deren Steuergerät handeln. Typischerweise weist ein ophthalmologisches Lasertherapiesystem eine Einheit zur elektromotorischen Verstellung des Laserstrahls und damit des Wechselwirkungsortes auf; auch diese Einheit stellt ein elektrisches Lastelement dar. All diese Komponenten verbrauchen im Betrieb des ophthalmologischen Lasertherapiesystems typischerweise elektrische Leistung, wobei sich die Leistungsaufnahme aufteilen lässt in einen Anteil, der einen quasi konstanten Grundverbrauch darstellt, und einen variablen Anteil. Der variable Anteil der Leistungsaufnahme kann beispielsweise während einer Laserbehandlung deutlich anwachsen und somit erhebliche Leistungsschwanken verursachen. Durch starke Leistungsschwankungen ergeben sich spezielle Anforderungen an eine Energieversorgung der Lastelemente.

Bei der Auslegung eines ophthalmologischen Lasertherapiesystems ist zu beachten, dass grundsätzlich die zur Verfügung stehende elektrische Leistung begrenzt ist. Typischerweise ist dabei der Ort, an dem das Lasersystem aufgestellt werden soll, der limitierende Faktor. Im Detail haben Sicherungswerte, Kabelquerschnitte und Steckertypen Einfluss auf die maximal verfügbare elektrische Leistung. Weiterhin wird die zur Verfügung stehende Leistung durch die lokale elektrische Netzspannung (in Volt, V) des lokalen Stromnetzes begrenzt, welche weltweit zwischen 100V (beispielsweise in Japan) und 240V (wie etwa in Europa) liegt.

Um ein elektrisches Gerät weltweit vertreiben und nutzen zu können, ohne auf lokale Besonderheiten der Stromversorgung Rücksicht nehmen zu müssen, werden typischerweise Weitbereichsnetzteile und wechselbare Netzkabel mit landestypischem Stecker verwendet. Durch die Verwendung von Weitbereichsnetzteilen wird eine Funktion des Gerätes weltweit sichergestellt, da trotz Bereitstellung unterschiedliche lokale Netzspannungen die Energieversorgung des Gerätes gewährleistet bleibt. Unter einen Weitbereichsnetzteil ist also ein Netzteil zu verstehen, das einen Weitbereichseingang aufweist, über den das Netzteil mit allen weltweit üblichen Netzspannungen betreibbar ist. Auf diese Weise ist weltweit eine einfache Installation des Gerätes möglich, ohne dass die Installation durch einen Fachmann für elektrische Schaltungsanlagen erforderlich ist. Dies ist insbesondere dann vorteilhaft, wenn ein und dasselbe Gerät an unterschiedlichen Orten eingesetzt werden soll. Um eine maximale Eingangsleistung für das Gerät erreichen zu können, sind Netzkabel zu verwenden, die einen maximalen Strom erlauben. Bei Verwendung von genormten Netzkabeln kann dies beispielsweise mit dem Steckertyp IEC-60320 C19/C20 (Kaltgeräte Stecker für 16 Ampere, A) ermöglicht werden. Soll das Gerät weltweit eingesetzt werden können, ergibt sich als Minimum einer maximale Leistungsaufnahme (in Watt, W) ein Wert von 100V * 16A = 1600W. Unter Berücksichtigung von 10% Toleranz in der Netzspannung, einer zwingenden Einhaltung des Maximalstroms von 16A (zur Sicherungsauslösung, wegen Kabelzertifizierung, ...) und einem typischen Wirkungsgrad des Netzteils von etwa 80-90% ergeben sich etwa 1300W elektrische Nutzleistung für das Gerät. Bei großen Phasenwinkel kann es zu einer weiteren Reduzierung der Nutzleistung kommen.

Im Falle von medizinischen Geräten und insbesondere von einem ophthalmologischen Lasertherapiesystem ist es vorteilhaft, auf den Einsatz von nicht-demontierbaren elektrischen Anschlüssen (z.B. für Drehstrom) zu verzichten, um das medizinische Gerät ohne einen Fachmann weltweit und räumlich flexibel (z.B. an verschiedenen Orten in einer Klinik) installieren zu können. Weiterhin ist es vorteilhaft, keine länderspezifische Konfektionierung des medizinischen Gerätes (und insbesondere von dessen Netzteil) mit einem fest an das Gerät angeschlossenen Netzkabel vorzunehmen, da dies zu einem erhöhten Produktions- und/oder Serviceaufwand führen kann. Unter diesen Randbedingungen ist es also wünschenswert, ein medizinisches Gerät derart auszuführen, dass es mit einer Leistungsaufnahme von lediglich 1600W voll funktionsfähig ist.

Im Detail bedeutet dies, dass ein Leistungsbudget für die unterschiedlichen Komponenten (elektrischen Lastelemente) des medizinischen Gerätes vergeben werden muss, um die oben genannte Bedingung einer maximalen Leistungsaufnahme zu erfüllen. Bei den elektrischen Lastelementen, insbesondere solchen mit einem hohen Leistungsbedarf, kann es sich bei einem ophthalmologischen Lasertherapiesystem um eine Laserquelle zur Erzeugung eines Therapiestrahls, um eine Scaneinheit zur Strahlablenkung des Laserstrahls, einen Computer, einen Monitor oder beispielsweise eine Verstelleinheit zur Ausrichtung des Patienten gegenüber dem Lasersystem handeln.

Aufgrund der begrenzten maximalen Leistungsaufnahme ist es eine besondere Herausforderung, trotz Leistungsschwanken, die während des Betriebs des medizinischen Gerätes auftreten können, seine Funktionalität sicherzustellen. In der Schrift US 2008/0269729 A1 sind ein Verfahren und eine Vorrichtung vorgeschlagen, die einen sicheren Gebrauch eines ophthalmologischen Lasertherapiesystems bei einer extremen Leistungsschwankung sicherstellt, nämlich bei Zusammenbruch der Stromversorgung des Systems. Dazu weist das Lasersystem eine wiederaufladbare Batterie auf, deren Kapazität so groß ist, im Fall einer Unterbrechung der Stromversorgung eine Durchführung der lasertherapeutischen Behandlung zu gewährleisten. In der genannten Schrift ist jedoch keine Lösung gezeigt, wie bei einem Auftreten von Leistungsspitzen, die beispielsweise während des eigentlichen therapeutischen Eingriffs auftreten können, eine Funktionalität des Lasersystems sichergestellt werden kann.

In Dokument KR 2016 0008313 A ist eine Schutzvorrichtung für periphere Geräte in Hochenergie-Lasersystemen beschrieben.

Aufgabe der vorliegenden Erfindung ist es deshalb, die Funktionsfähigkeit eines ophthalmologischen Lasertherapiesystems mit einer begrenzten Leistungsaufnahme bei einem Auftreten von Leistungsspitzen sicherzustellen. Erfindungsgemäß wird die Aufgabe durch die Merkmale des unabhängigen Anspruchs gelöst. Bevorzugte Weiterbildungen und Ausgestaltungen sind Gegenstand der abhängigen Ansprüche.

Die Erfindung betrifft ein ophthalmologisches Lasertherapiesystem. Das ophthalmologische Lasertherapiesystem weist eine Leistungsaufnahme von bis zu 1600W auf sowie ein elektrisches Lastelement und einen Zwischenspeicher.

Ein ophthalmologisches Lasertherapiesystem ist dazu ausgebildet, bei einer Verwendung durch einen Benutzer (Operateur, Chirurgen, Bediener) eine ophthalmologische Therapie mittels Laserstrahlung durchzuführen. Unter einer ophthalmologischen Therapie soll dabei jegliche Therapie verstanden werden, bei der ein Gewebe eines Auges, insbesondere der Cornea, verändert wird. Die ophthalmologische Therapie beinhaltet insbesondere entsprechende laserchirurgische Eingriffe, bei denen mittels eines Lasers, bevorzugt eines gepulsten Lasers wie beispielsweise eines Femtosekunden- oder Excimer-Lasers, durch Photodisruption ein Gewebe des Auges "geschnitten" wird (z.B. ein Lentikel oder ein Flap), durch eine Ablationswirkung ein Bereich eines Augengewebes abgetragen wird oder durch eine Koagulationswirkung Augengewebe miteinander "verklebt" wird bzw. der Brechungsindex des Materials, also eines Augengewebes oder aber eines Implantats, durch die Laserstrahlung verändert wird.

Ein ophthalmologisches Lasertherapiesystem wird im Rahmen der vorliegenden Offenbarung auch als "Lasersystem" bezeichnet.

Das ophthalmologische Lasertherapiesystem weist dabei eine Leistungsaufnahme von bis zu 1600W auf. Dazu kann das Lasersystem ein Weitbereichsnetzteile und ein (wechselbares) Netzkabel mit landestypischem Stecker aufweisen. Bei dem Netzkabel kann es sich beispielsweise um ein genormtes Netzkabel handeln, das für einen Strom von 16A ausgelegt ist. Unter Berücksichtigung von Toleranzen in der Netzspannung und einem typischen Wirkungsgrad von Netzteilen stehen dem Lasersystem somit etwa 1300W elektrische Nutzleistung zur Verfügung.

Weiterhin weist das ophthalmologische Lasertherapiesystem ein elektrisches Lastelement auf. Ein elektrisches Lastelement wird im Rahmen der Offenbarung auch lediglich als "Lastelement" bezeichnet. Das Lastelement kann dabei eine oder mehrere elektrische Komponenten aufweisen. Mit anderen Worten stellt ein elektrisches Lastelement einen Verbraucher von elektrischer Energie dar oder weist einen oder mehrere solche Verbraucher auf.

Bei einem elektrischen Lastelement kann es sich beispielsweise um eine Lasereinheit zur Bereitstellung von therapeutischer Laserstrahlung handeln. Es kann sich auch um eine Steuereinheit (auch Steuergerät genannt) zur Steuerung von elektrischen Lastelementen des Lasersystems handeln. Es kann sich um eine Recheneinheit (Computer mit CPU und Speicher) handeln oder um einen Monitor. Das Lasersystem kann auch eine Positioniereinheit, die typischerweise Aktoren umfasst, zur Ausrichtung eines Patienten gegenüber dem Lasersystem als elektrisches Lastelement aufweisen. Bei dem Lastelement kann es sich auch um eine Strahlversatzeinheit zur elektromotorischen, räumlichen Verstellung des Laserstrahls und damit des Wechselwirkungsortes (in zwei oder drei Raumdimensionen) handeln. Die Strahlversatzeinheit kann dazu einen oder mehrere Scanner sowie eine Scannersteuerung aufweisen.

Erfindungsgemäß ist der Zwischenspeicher dazu ausgebildet, dem elektrischen Lastelement zyklisch eine Energie von mindestens 30mJ (Milli-Joule) bereitzustellen, vorzugsweise mindestens 40mJ, besonders bevorzugt mindestens 50mJ. Unter einer zyklischen Bereitstellung ist dabei zu verstehen, dass mehrfach (mindestens 10fach, bevorzugt mindestens 100fach, besonders bevorzugt mindestens 1000fach) eine Energie für einen Verbrauch durch das Lastelement verfügbar ist. Dabei muss die Frequenz der Bereitstellung der Energie nicht konstant über die Anzahl der Bereitstellungen sein. Die Frequenz kann sich beispielsweise mit der Zeit stetig, linear oder mit einer (ganzzahligen oder nicht-ganzzahligen) Potenz in der Zeit ändern.

Durch die zyklische Bereitstellung von Energie können zyklisch auftretende Leistungsspitzen durch einen Leistungsabruf des Lastelements des Lasersystems abgefangen, gepuffert oder geglättet werden, ohne dass die maximale Leistungsaufnahme des Lasersystems überschritten wird. Dadurch kann auch für solche Leistungsspitzen der reibungslose Ablauf eines lasertherapeutischen Eingriffs sichergestellt werden.

In einer vorteilhaften Ausgestaltung ist der Zwischenspeicher dazu ausgebildet, dem elektrischen Lastelement zyklisch eine Energie von bis zu 220mJ bereitzustellen, vorzugsweise bis zu 200mJ, besonders bevorzugt bis zu 180mJ.

Gemäß einer weiteren Ausgestaltung des Zwischenspeichers ist dieser dazu ausgebildet, die Energie zyklisch mit einer Frequenz von mindestens 20Hz bereitzustellen, bevorzugt mit mindestens 40Hz, besonders bevorzugt mit mindestens 60Hz. Zusätzlich oder alternativ ist der Zwischenspeichers dazu ausgebildet, die Energie zyklisch mit einer Frequenz von bis zu 6kHz bereitzustellen, bevorzugt mit bis zu 5.5kHz, besonders bevorzugt mit bis zu 5kHz.

Handelt es sich bei dem elektrischen Lastelement um die Strahlversatzeinheit, so könnte diese beispielsweise die Laserstrahlung zyklisch mit mindestens 10Hz (bzw. mindestens 20Hz oder 30Hz) ablenken und/oder mit bis zu 3kHz (bzw. 2.75kHz oder 2.5kHz); die Halbierung der Frequenzen ergibt sich dadurch, dass innerhalb einer Scan-Periode der Strahlversatzeinheit zwei Leistungsspitzen auftreten können.

Durch die genannten Frequenzbereiche lassen sich vorteilhaft Leistungsspitzen, die beispielsweise durch die Strahlversatzeinheit beim Scannen des Laserstrahls im Auge - um beispielsweise ein Lentikel oder ein Flap zu schneiden - hervorgerufen werden, derart abfangen oder puffern, dass die maximale Leistungsaufnahme des Lasersystems nicht überschritten wird.

Dadurch kann auch für solche Leistungsspitzen der reibungslose Ablauf eines lasertherapeutischen Eingriffs sichergestellt werden.

Gemäß einer besonders vorteilhaften Ausgestaltung des Zwischenspeichers weist dieser einen Kondensator und/oder eine Batterie auf. Auf diese Weise werden schnelle Umladungsprozesse, also eine schnelle Abgabe von Energie, ermöglicht, wie sie beispielsweise bei einer zyklischen Bereitstellung von Energie im Kilohertz-Bereich erforderlich ist. Auch hohe Leistungsspitzen können auf diese Weise geglättet werden. Ein Kondensator weist dazu vorteilhaft eine hohe Kapazität auf, um kurzzeitig einen hohen Stromfluss gewährleisten zu können. Es kann sich bei dem Kondensator um einen Elektrolytkondensator oder einen sogenannten Super-Cap handeln. Eine Batterie weist vorteilhafterweise einen geringen Innenwiderstand auf. Dieser kann beispielsweise geringer als 0.1 Ohm sein oder bis zu 0.001 Ohm betragen. Bei Batterien mit geringem Innenwiederstand werden nicht so hohe Kapazitäten benötigt, da die Spannung unter Last nicht so schnell zusammenbricht. Es kann sich bei der Batterie beispielsweise um einen sogenannten Hochstom-Akku (z.B.LiPo-Akku) handeln.

In einer besonders bevorzugten Ausgestaltung ist der Zwischenspeicher dadurch gekennzeichnet, dass die zyklische Bereitstellung von Energie für ein Lastelement erfolgt, das einen zyklischen Leistungsbedarf aufweist. Während einer lasertherapeutischen Behandlung kann ein zyklischer Leistungsbedarf zu zyklischen Leistungsspitzen führen. Bei einem Lastelemente mit einem zyklischen Leistungsbedarf kann es sich beispielsweise um die Lasereinheit zur Bereitstellung von therapeutischer Laserstrahlung handeln. Bei dem Lastelement kann es sich auch um die Strahlversatzeinheit zur elektromotorischen, räumlichen Verstellung des Laserstrahls und damit des Wechselwirkungsortes (in zwei oder drei Raumdimensionen) handeln. Die Strahlversatzeinheit kann dazu einen oder mehrere Scanner aufweisen sowie eine Scannersteuerung. Vorteilhaft ist der Zwischenspeicher dazu ausgebildet, dem Lastelement die Energie mit einer Frequenz bereitzustellen, die der Frequenz des zyklischen Leistungsbedarfs entspricht.

In einer bevorzugten Ausgestaltung des ophthalmologischen Lasertherapiesystems weist dieses mindestens ein weiteres elektrischen Lastelement sowie eine Energieversorgungseinheit auf. Dabei ist die Energieversorgungseinheit mit dem elektrischen Lastelement und dem mindestens einen weiteren elektrischen Lastelement derart elektrisch verbunden, dass eine Versorgung mit Energie ermöglicht wird. Die Energieversorgungseinheit verteilt gewissermaßen die Energie auf die mindestens zwei Lastelemente. Dabei kann der Energieversorgungseinheit die gesamte aufgenommene Leistung des Lasersystems von 1600W zur Verfügung stehen.

Erfindungsgemäß ist der Zwischenspeicher derart mit der Energieversorgungseinheit und dem elektrischen Lastelement elektrisch verbunden, dass nur dem elektrischen Lastelement die Energie zyklisch bereitgestellt wird. Mit anderen Worten wird nur dem elektrischen Lastelement, dessen zyklische Leistungsspitzen gepuffert werden sollen, zyklisch die dazu erforderliche Energie bereitgestellt, während weitere elektrische Lastelemente davon ausgenommen werden. Auf diese Weise kann vorteilhaft sichergestellt werden, dass eine "Glättung" von Leistungsspitzen des elektrischen Lastelementes sich nicht negativ auf weitere elektrische Lastelemente auswirken. In einem Stromnetzsystem des Lasersystems erfolgt somit gewissermaßen die Pufferung der benötigten Leistung durch den Zwischenspeicher am Ort der variablen Leistungsentnahme.

In einer Variante des ophthalmologischen Lasertherapiesystems, vorzugsweise nach einer der oben beschriebenen Ausgestaltungen, mit einer Leistungsaufnahme von bis zu 1600W weist dieses mindestens zwei elektrische Lastelemente, eine Steuereinheit und vorzugsweise einen Zwischenspeicher nach einer der vorgenannten Ausgestaltungen auf. Dabei ist die Steuereinheit erfindungsgemäß derart ausgestaltet, dass die elektrischen Lastelemente sequenziell ein- und ausgeschaltet werden können. Durch ein sequenzielles Ein- bzw. Ausschalten der Lastelemente kann eine Akkumulation von Leistungsspitzen vermieden werden, die sich als Summe oder Überlagerung von Leistungsspitzen der einzelnen Lastelemente ergibt. Des Weiteren können auf diese Weise elektrische Lastelemente, die beispielsweise während einer Phase des ophthalmologischen Eingriffs nicht benötigt werden, ausgeschaltet werden, um temporär die benötigte Gesamtleistung zu verringern.

Gemäß einer Ausgestaltung des ophthalmologischen Lasertherapiesystems ist diese dadurch gekennzeichnet, dass es eine Energieversorgungseinheit aufweist, die als Weitbereichsnetzteil ausgebildet ist. Weiterhin umfasst das ophthalmologische Lasertherapiesystem ein wechselbares Netzkabel, über das die Energieversorgungseinheit mit einem lokalen Stromnetz elektrisch verbunden werden kann. Auf diese Weise wird es möglich, das ophthalmologische Lasertherapiesystem weltweit vertreiben und nutzen zu können, ohne auf lokale Besonderheiten der Stromversorgung Rücksicht nehmen zu müssen. Gleichzeitig wird über den Zwischenspeicher sichergestellt, dass Leistungsspitzen im Betrieb des Gerätes gepuffert werden können, obwohl die Leistungsaufnahme lediglich bis zu 1600W beträgt.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, näher erläutert. Es zeigen:
Fig. 1 eine schematische Darstellung eines beispielhaften ophthalmologischen Lasertherapiesystems mit einem Zwischenspeicher;
Fig. 2 eine Darstellung des schematischen Aufbaus eines erfindungsgemäßen ophthalmologischen Lasertherapiesystem mit Zwischenspeicher.

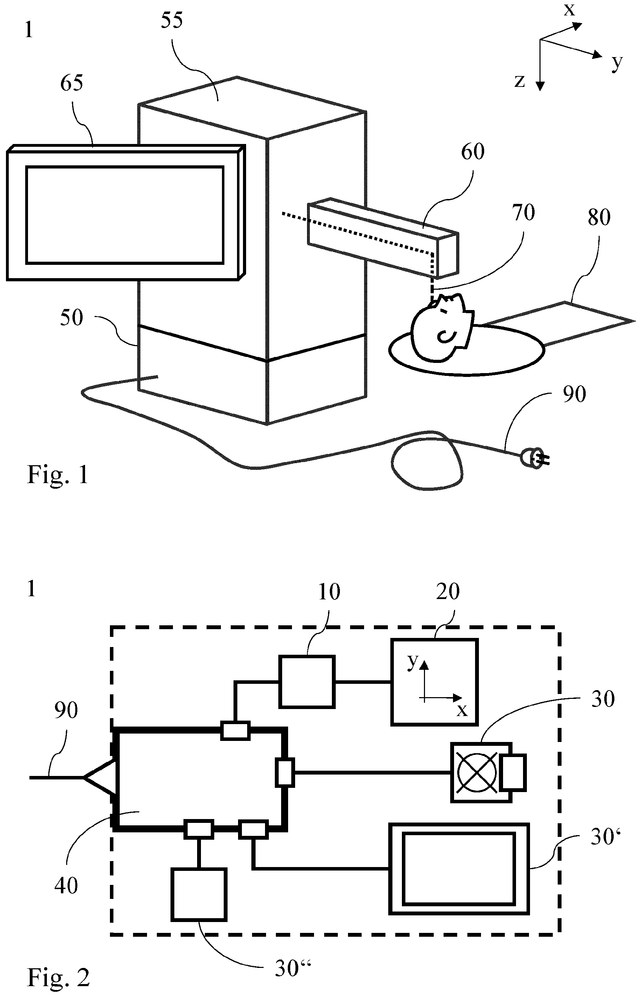

In Fig. 1 ist eine schematische Darstellung eines beispielhaften ophthalmologischen Lasertherapiesystems 1 gezeigt.

Das Beispiel des ophthalmologischen Lasertherapiesystems 1 setzt sich zusammen aus einer Gerätebasis 50 und einem auf dieser Gerätebasis 50 in der Höhe über einer Bodenebene, also der z-Richtung, und in seiner Position in der Ebene, also in x- und y-Richtung, verstellbaren Gerätekopf 55. Der Gerätekopf 55 umfasst im gezeigten Beispiel eine zur Erzeugung eines entsprechenden gepulsten Laserstrahls erforderliche Lasereinheit (nicht dargestellt), die hier eine Femtosekunden-Laserquelle ist. Die von der Laserquelle erzeugte therapeutische Laserstrahlung 70 wird über einen Laserarm 60 zum Therapieort im Auge eines Patienten geleitet. Die therapeutische Laserstrahlung 70 ist innerhalb des Lasersystems 1 gepunktet dargestellt, außerhalb davon durch eine gestrichelte Linie. Zur elektromotorischen, räumlichen Verstellung des Laserstrahls und damit des Wechselwirkungsortes (Therapieortes im Auge des Patienten) weist der Gerätekopf 55 eine Strahlversatzeinheit auf (nicht eingezeichnet). Im gezeigten Beispiel weist die Strahlversatzeinheit ein Paar von Scannern auf, die eine Ablenkung der therapeutischen Laserstrahlung 70 am Therapieort in der x-y-Ebene erlauben.

Zur Ausrichtung des Patienten gegenüber dem ophthalmologischen Lasertherapiesystem 1 kann nicht nur über eine Bewegung des Gerätekopfes 55 erfolgen. Zusätzlich weist das Lasersystem als Positioniereinheit eine Patientenliege 80 auf, die Aktoren umfasst, und die ebenfalls eine Bewegung des Patienten gegenüber dem Lasersystem 1 erlaubt.

Weiterhin weist das ophthalmologische Lasertherapiesystem 1 einen Monitor 65 auf. Dieser erlaubt dem Benutzer des Lasersystems 1 die Überwachung und Steuerung einer lasertherapeutischen Behandlung. Die für eine Steuerung zusätzlich benötigten Bedieneinheiten (Tastatur, Joystick) sind nicht eingezeichnet.

Die Energieversorgung des ophthalmologische Lasertherapiesystems 1 erfolgt über ein Netzkabel 90. Dabei handelt es sich um ein genormtes Netzkabel mit einem Stecker vom Steckertyp IEC-60320 C19/C20. Über das Netzkabel 90 kann dem Lasersystem 1 ein Strom von 16A bereitgestellt werden. Dazu ist das Netzkabel 90 mit einem Weitbereichsnetzteile (nicht eingezeichnet) verbunden. Dabei ist die Verbindung so ausgestaltet, dass ein Austausch des Netzkabels 90 gegen ein anderes Netzkabel 90 mit einem anderen Stecker ermöglicht wird (ohne Servicetechniker). Das Weitbereichsnetzteil kann Spannungen von 100V bis 240V verarbeiten und eine Energieversorgung der verschiedenen Lastelemente (Verbraucher) entsprechend der jeweils benötigten Spannung sicherstellen. Wird über das lokale Stromnetz eine Spannung von 100V bereitgestellt, so beträgt die Leistungsaufnahme des ophthalmologische Lasertherapiesystems 1 bis zu 1600W.

Erfindungsgemäß weist das Lasersystem 1 einen Zwischenspeicher (nicht eingezeichnet) auf. Dieser ist einerseits mit dem Weitbereichsnetzteil und andererseits mit der Strahlversatzeinheit elektrisch verbunden. Bei der Strahlversatzeinheit handelt es sich hier um das elektrische Lastelement, das während einer lasertherapeutischen Behandlung zyklisch (in Abhängigkeit der Schwingungsfrequenz der Scanner) einen hohen Leistungsbedarf hat und somit Leistungsspitzen aufweist. Der Zwischenspeicher ist erfindungsgemäß dazu ausgebildet, diese zyklische auftretenden Leistungsspitzen durch eine Bereitstellung von Energie zu glätten.

In Fig. 2 ist eine Darstellung des schematischen Aufbaus eines erfindungsgemäßen ophthalmologischen Lasertherapiesystem 1 mit Zwischenspeicher 10 gezeigt. Über ein Netzkabel 90 ist das Lasersystem 1 mit dem lokalen Stromnetz (nicht eingezeichnet) elektrisch verbunden. Die lösbare elektrische Verbindung ist über ein dreieckiges Symbol am Netzkabel 90 dargestellt. Das Netzkabel 90 führt Energie der Energieversorgungseinheit 40 zu, die als Weitbereichsnetzteil ausgebildet ist. Dieses versorgt mehrere Verbraucher des Lasersystems 1 mit Energie. Bei der Strahlversatzeinheit handelt es sich um ein elektrisches Lastelement 20, welches während einer lasertherapeutischen Behandlung zyklisch einen hohen Leistungsbedarf hat und somit Leistungsspitzen aufweist. Um diese Leistungsspitzen zu glätten, ist das elektrische Lastelement 20 mit dem Zwischenspeicher 10 elektrisch verbunden, der wiederum mit der Energieversorgungseinheit 40 elektrisch verbunden ist. Der Zwischenspeicher ist erfindungsgemäß dazu ausgebildet, diese zyklische auftretenden Leistungsspitzen zu glätten.

Zusätzlich weist das ophthalmologischen Lasertherapiesystem 1 weitere elektrische Lastelemente 30, 30' bzw. 30" auf, welche im Betrieb keine zyklischen Leistungsspitzen verursachen, die zu glätten sind. Dabei handelt es sich hier um eine Lasereinheit, einen Monitor bzw. eine Steuereinheit. In der dargestellten Anordnung ist der Zwischenspeicher 10 derart mit der Energieversorgungseinheit 40 und dem elektrischen Lastelement 20, das im Betrieb zyklische Leistungsspitzen verursachen kann, elektrisch verbunden, dass nur dem Lastelement 20 die Energie zyklisch bereitgestellt wird, während die weiteren elektrischen Lastelemente 30, 30' und 30" von der Glättung der Leistungsspitzen nicht beeinflusst werden.

Die vorstehend genannten und in verschiedenen Ausführungsbeispielen beschriebenen Merkmale der Erfindung sind dabei nicht nur in den angegebenen beispielhaften Kombinationen, sondern auch in anderen Kombinationen oder allein einsetzbar, ohne den Rahmen der vorliegenden Erfindung zu verlassen.

## Patentansprüche

1. Ophthalmologisches Lasertherapiesystem (1), das ein elektrisches Lastelement (20) und einen Zwischenspeicher (10) aufweist, **dadurch gekennzeichnet, dass** das ophthalmologische Lasertherapiesystem (1) eine Leistungsaufnahme von bis zu 1600 Watt aufweist und dass der Zwischenspeicher (10) dazu ausgebildet ist, dem elektrischen Lastelement (20) zyklisch eine Energie von mindestens 30mJ bereitzustellen, vorzugsweise mindestens 40mJ, besonders bevorzugt mindestens 50mJ.

2. Ophthalmologisches Lasertherapiesystem (1) nach Anspruch 1, **dadurch gekennzeichnet, dass** der Zwischenspeicher (10) dazu ausgebildet ist, dem elektrischen Lastelement (20) zyklisch eine Energie von bis zu 220mJ bereitzustellen, vorzugsweise bis zu 200mJ, besonders bevorzugt bis zu 180mJ.

3. Ophthalmologisches Lasertherapiesystem (1) nach einem der vorgenannten Ansprüche, **dadurch gekennzeichnet, dass** die zyklische Bereitstellung mit einer Frequenz von mindestens 20Hz erfolgt, vorzugsweise mindestens 40Hz, besonders bevorzugt mindestens 60Hz, und/oder dass die zyklische Bereitstellung mit einer Frequenz von bis zu 6kHz erfolgt, vorzugsweise bis zu 5.5kHz, besonders bevorzugt bis zu 5kHz.

4. Ophthalmologisches Lasertherapiesystem (1) nach einem der
vorgenannten Ansprüche, **dadurch gekennzeichnet, dass** der Zwischenspeicher (10) einen Kondensator und/oder eine Batterie aufweist.

5. Ophthalmologisches Lasertherapiesystem (1) nach einem der vorgenannten Ansprüche, **dadurch gekennzeichnet, dass** die zyklische Bereitstellung von Energie für das Lastelement (20) erfolgt, das einen zyklischen Leistungsbedarf aufweist.

6. Ophthalmologisches Lasertherapiesystem (1) nach einem der vorgenannten Ansprüche, **dadurch gekennzeichnet, dass** das Lastelement (20) eine Strahlversatzeinheit umfasst, die vorzugsweise einen Scanner und eine Scannersteuerung aufweist.

7. Ophthalmologisches Lasertherapiesystem (1) nach einem der vorgenannten Ansprüche, **dadurch gekennzeichnet, dass** das ophthalmologisches Lasertherapiesystem (1) mindestens ein weiteres elektrisches Lastelement (30, 30', 30") und eine Energieversorgungseinheit (40) aufweist, wobei die Energieversorgungseinheit (40) mit dem elektrischen Lastelement (20) und dem mindestens einen weiteren elektrischen Lastelement (30, 30', 30") derart elektrisch verbunden ist, dass eine Versorgung mit Energie ermöglicht wird, und wobei der Zwischenspeicher (10) derart mit der Energieversorgungseinheit (40) und dem elektrischen Lastelement (20) elektrisch verbunden ist, dass nur dem elektrischen Lastelement (20) die Energie zyklisch bereitgestellt wird.

8. Ophthalmologisches Lasertherapiesystem (1) nach einem der vorgenannten Ansprüche, **dadurch gekennzeichnet, dass** das ophthalmologisches Lasertherapiesystem (1) eine Energieversorgungseinheit (40), die als Weitbereichsnetzteil ausgebildet ist, und ein wechselbares Netzkabel (90) aufweist, über das die Energieversorgungseinheit (40) mit einem lokalen Stromnetz elektrisch verbunden werden kann.

## Claims

1. Ophthalmological laser therapy system (1) having an electrical load element (20) and an intermediate storage unit (10),
**characterized in that** the ophthalmological laser therapy system (1) has a power consumption of up to 1600 watts, and **in that** the intermediate storage unit (10) is configured to provide cyclically an energy of at least 30 mJ, preferably at least 40 mJ, particularly preferably at least 50 mJ, for the electrical load element (20).

2. Ophthalmological laser therapy system (1) according to Claim 1, **characterized in that** the intermediate storage unit (10) is configured to provide cyclically an energy of up to 220 mJ, preferably up to 200 mJ, particularly preferably up to 180 mJ, for the electrical load element (20).

3. Ophthalmological laser therapy system (1) according to either of the preceding claims, **characterized in that** the cyclical provision is effected at a frequency of at least 20 Hz, preferably at least 40 Hz, particularly preferably at least 60 Hz, and/or **in that** the cyclical provision is effected at a frequency of up to 6 kHz, preferably up to 5.5 kHz, particularly preferably up to 5 kHz.

4. Ophthalmological laser therapy system (1) according to any of the preceding claims, **characterized in that** the intermediate storage unit (10) has a capacitor and/or a battery.

5. Ophthalmological laser therapy system (1) according to any of the preceding claims, **characterized in that** the cyclical provision of energy is effected for the load element (20) having a cyclical power demand.

6. Ophthalmological laser therapy system (1) according to any of the preceding claims, **characterized in that** the load element (20) comprises a beam offset unit, which preferably has a scanner and a scanner controller.

7. Ophthalmological laser therapy system (1) according to any of the preceding claims, **characterized in that** the ophthalmological laser therapy system (1) has at least one further electrical load element (30, 30', 30") and an energy supply unit (40), the energy supply unit (40) being electrically connected to the electrical load element (20) and the at least one further electrical load element (30, 30', 30") in such a way that a supply with energy is made possible, and the intermediate storage unit (10) being electrically connected to the energy supply unit (40) and the electrical load element (20) in such a way that the energy is provided cyclically only for the electrical load element (20).

8. Ophthalmological laser therapy system (1) according to any of the preceding claims, **characterized in that** the ophthalmological laser therapy system (1) has an energy supply unit (40) configured as a wide-range power supply unit, and an interchangeable power supply system cable (90), via which the energy supply unit (40) can be electrically connected to a local power supply system.

## Revendications

1. Système de thérapie ophtalmologique par laser (1) comportant un élément de charge électrique (20) et un accumulateur intermédiaire (10),
**caractérisé en ce que** le système de thérapie ophtalmologique par laser (1) présente une puissance absorbée allant jusqu'à 1600 watts et **en ce que** l'accumulateur intermédiaire (10) est conçu pour fournir cycliquement à l'élément de charge électrique (20) une énergie d'au moins 30 mJ, de préférence d'au moins 40 mJ, et de manière particulièrement préférée d'au moins 50 mJ.

2. Système de thérapie ophtalmologique par laser (1) selon la revendication 1, **caractérisé en ce que** l'accumulateur intermédiaire (10) est conçu pour fournir cycliquement à l'élément de charge électrique (20) une énergie allant jusqu'à 220 mJ, de préférence jusqu'à 200 mJ, et de manière particulièrement préférée jusqu'à 180 mJ.

3. Système de thérapie ophtalmologique par laser (1) selon l'une quelconque des revendications précédentes, **caractérisé en ce que** la fourniture cyclique s'effectue à une fréquence d'au moins 20 Hz, de préférence d'au moins 40 Hz, et de manière particulièrement préférée d'au moins 60 Hz, et/ou **en ce que** la fourniture cyclique s'effectue à une fréquence allant jusqu'à 6 kHz, de préférence jusqu'à 5,5 kHz, et de manière particulièrement préférée jusqu'à 5 kHz.

4. Système de thérapie ophtalmologique par laser (1) selon l'une quelconque des revendications précédentes, **caractérisé en ce que** l'accumulateur intermédiaire (10) comporte un condensateur et/ou une batterie.

5. Système de thérapie ophtalmologique par laser (1) selon l'une quelconque des revendications précédentes, **caractérisé en ce que** la fourniture cyclique d'énergie s'effectue pour l'élément de charge (20), lequel présente un besoin cyclique en énergie.

6. Système de thérapie ophtalmologique par laser (1) selon l'une quelconque des revendications précédentes, **caractérisé en ce que** l'élément de charge (20) comprend une unité de décalage de faisceau, laquelle comporte de préférence un scanner et un dispositif de commande de scanner.

7. Système de thérapie ophtalmologique par laser (1) selon l'une quelconque des revendications précédentes, **caractérisé en ce que** le système de thérapie ophtalmologique par laser (1) comporte au moins un autre élément de charge électrique (30, 30', 30") et une unité d'alimentation en énergie (40), l'unité d'alimentation en énergie (40) étant reliée électriquement à l'élément de charge électrique (20) et à au moins un autre élément de charge électrique (30, 30', 30") de manière à permettre une alimentation en énergie, et l'accumulateur intermédiaire (10) étant relié électriquement à l'unité d'alimentation en énergie (40) et à l'élément de charge électrique (20) de telle sorte que l'énergie n'est fournie cycliquement qu'à l'élément de charge électrique (20).

8. Système de thérapie ophtalmologique par laser (1) selon l'une quelconque des revendications précédentes, **caractérisé en ce que** le système de thérapie ophtalmologique par laser (1) comporte une unité d'alimentation en énergie (40), laquelle est réalisée sous la forme d'une alimentation en énergie à large plage, ainsi qu'un câble d'alimentation en énergie (90) interchangeable, par l'intermédiaire duquel l'unité d'alimentation en énergie (40) peut être reliée électriquement à un réseau électrique local.
